Europäisches Patentamt

European Patent Office

Office européen des brevets

(11) Numéro de publication: **0 243 213**
**A1**

# (12) DEMANDE DE BREVET EUROPEEN

(21) Numéro de dépôt: **87400222.3**

(22) Date de dépôt: **02.02.87**

(51) Int. Cl.⁴: **A 61 L 2/06**
**A 23 L 3/14**

(30) Priorité: **14.02.86 FR 8602046**

(43) Date de publication de la demande:
**28.10.87 Bulletin 87/44**

(84) Etats contractants désignés: **DE ES GB IT SE**

(71) Demandeur: **BARRIQUAND, Société dite:**
**9 à 13 Rue Saint Claude**
**F-42300 Roanne (FR)**

(72) Inventeur: **Drouin, Patrice**
**37 rue F. Buisson**
**F-42300 Roanne (FR)**

**Roumagnac, Jean-Patrick**
**25 rue A. Gelin**
**F-42120 Le Coteau (FR)**

(74) Mandataire: **Cianet, Denis et al**
**Cabinet Beau de Loménie 55, rue d'Amsterdam**
**F-75008 Paris (FR)**

(54) **Autoclave de stérilisation avec dispositif de ruissellement.**

(57) Autoclave de stérilisation (10) comprenant une cuve (12) dans laquelle est reçu au moins un conteneur (20) ajouré où sont placés des récipients (22) à stériliser, et au moins une rampe (24) d'aspersion située dans la cuve et destinée à la répartition d'un liquide de stérilisation, lequel se rassemble après ruissellement en partie basse (28) de la cuve, caractérisé en ce qu'il comprend un tambour (14) dans ladite cuve, monté rotatif autour d'un axe (XX) horizontal et recevant ledit conteneur (20), et que ladite rampe (24) s'étend dans un espace délimité entre le tambour (14) et la partie supérieure de la cuve.

FIG.1

EP 0 243 213 A1

**Description**

## AUTOCLAVE DE STERILISATION AVEC ISPOSITIF DE RUISSELLEMENT

La présente invention concerne un autoclave de stérilisation avec dispositif de ruissellement.

Un autoclave de ce type comprend une cuve dans laquelle est reçu au moins un conteneur ajouré où sont placés des récipients à stériliser.

Une ou plusieurs rampes d'aspersion dans la cuve sont destinées à la répartition d'un liquide de stérilisation, habituellement de l'eau à haute température, qui vient donc ruisseler sur les récipients à stériliser. Ce liquide se rassemble ensuite en partie basse de la cuve, d'où il est puisé par une pompe de recirculation qui le refoule vers les rampes via un échangeur de réchauffage et/ou de refroidissement.

Pour des raisons évidentes de construction, on prévoit plusieurs fixes afin que toutes les zones du conteneur soient également desservies en liquide de stérilisation.

Cependant, dans certaines applications, il est nécessaire de prévoir une agitation constante des récipients à stériliser en raison notamment du produit qu'ils contiennent.

Conformément à l'invention ce but est atteint grâce au fait que le conteneur est monté dans un tambour rotatif autour d'un axe horizontal, ledit tambour étant reçu dans ladite cuve et délimitant avec la partie supérieure de celle-ci un espace où s'étend la -ou les- rampe(s) d'aspersion.

Ainsi, grâce à l'invention, les récipients sont continuellement agités par la rotation du tambour, tout en recevant le liquide de stérilisation provenant de la -ou des- rampe(s) de stérilisation, étant fait observer que la rotation du tambour permet de prévoir une rampe d'aspersion unique sans perte d'efficacité tout en assurant que le ruissellement du liquide de stérilisation s'effectue dans toutes les zones du conteneur.

Avantageusement, le tambour comporte plusieurs goulottes, faisant office de godets, régulièrement réparties à sa périphérie, ouvertes sensiblement en direction de l'axe de rotation et traversant la partie basse de la cuve au cours de la rotation du tambour.

Grâce à cette disposition, les goulottes faisant office de godets se remplissent de liquide de stérilisation lors de leur passage en partie basse de la cuve, qu'elles déversent sur le conteneur lorsque la rotation du tambour les a élevées jusqu'à une hauteur telle que leur ouverture soit orientée vers le bas, ce qui complète avantageusement l'aspersion obtenue par la ou les rampe(s).

L'intensité de l'aspersion complémentaire peut être modifiée en fonction du nombre et de la taille des goulottes faisant office de godets.

De plus, le passage continuel des goulottes faisant office de godets dans le liquide de stérilisation recueilli en partie basse de la cuve assure un excellent brassage de celui-ci.

L'invention sera maintenant décrite à l'aide des dessins annexés, dans lequels :

La figure 1 est une vue en coupe schématique d'un autoclave de stérilisation conforme à la présente invention, et

la figure 2 est une vue en coupe axiale schématique de l'autoclave de la figure 1.

L'autoclave de stérilisation désigné par la référence générale 10 comprend une cuve cylindrique 12 d'axe horizontal, dans laquelle est monté un tambour 14, rotatif autour d'un axe horizontal XX. L'entraînement du tambour s'effectue au moyen d'un moteur 16, situé à l'extérieur de la cuve, et son supportage est assuré par deux rouleaux fous 18 placés en partie basse de la cuve.

En variante, non représentée, l'entraînement du tambour peut être assuré par l'intermédiaire des rouleaux-supports 18.

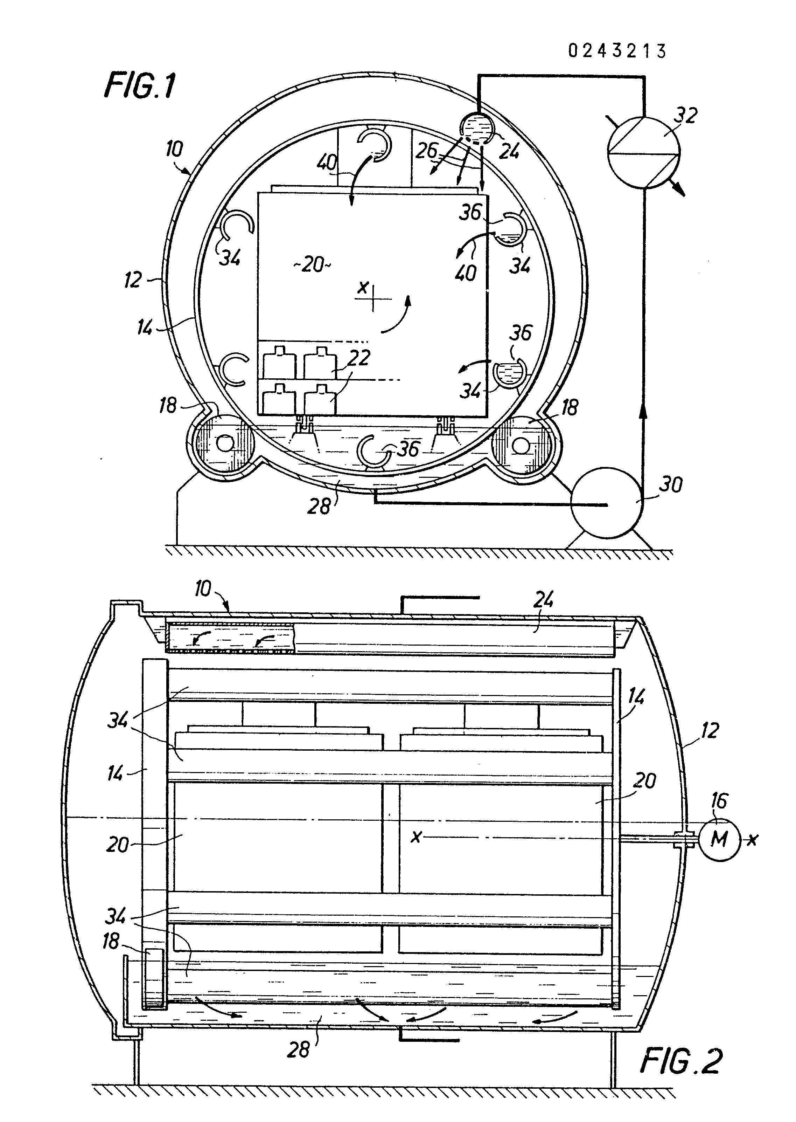

Le tambour reçoit un ou plusieurs conteneurs 20, au nombre de deux dans le mode de réalisation illustré (figure 2), dont les faces sont ajourées et où sont disposés des récipients 22 ou analogue à stériliser.

Une rampe d'aspersion 24, placée dans l'espace délimité entre le tambour et le sommet de la cuve, assure la répartition d'un liquide de stérilisation (flèche 26) qui vient donc ruisseler sur les récipients à stériliser. Grâce à la rotation du tambour, l'orientation du conteneur et des récipient par rapport à la rampe 24 varie continuellement, ce qui assure un ruissellement du liquide de stérilisation sur les récipients dans toutes les zones du conteneur.

Le liquide de stérilisation se rassemble en partie basse de la cuve (28) d'où il est puisé par une pompe 30 puis refoulé à nouveau par la rampe 24 après traversée d'un échangeur de réchauffage 32 et/ou refroidissement.

Le tambour comporte également plusieurs goulottes 34 faisant office de godets, régulièrement réparties à sa périphérie, et pourvues d'une ouverture 36 orientée sensiblement en direction de l'axe de rotation qui traversent la partie basse de la cuve au cours de la rotation du tambour.

Grâce à cette disposition les goulottes faisant office de godets se remplissent de liquide de stérilisation lors de leur traversée de la partie basse de la cuve, puis se déversent (flèches 40) sur le conteneur lorsqu'elles s'élèvent au fur et à mesure dela rotation du tambour.

En premier lieu, ceci assure une aspersion complémentaire du conteneur et des récipients qu'il contient, grâce à une disposition particulièrement simple.

En second lieu, le passage des goulottes faisant office de godets dans la partie basse de la cuve assure un brassage et une homogénéisation du liquide de stérilisation.

Naturellement, l'intensité de l'aspersion complémentaire assurée par les goulottes faisant office de godets est fonction du nombre et de la taille des goulottes, ainsi que de la vitesse de rotation du tambour. On pourra donc modifier l'un et/ou les autres de ces paramètres selon le résultat recherché pour une application déterminée.

Avantageusement, les goulottes faisant office de godets ont une forme semi-tubulaire et présentent en section un profil circulaire incomplet, la partie manquante formant l'ouverture 36 par laquelle la goulotte se remplit lors de la traversée de la partie basse de la cuve puis se déverse après sa remontée lors de la rotation du tambour.

Comme on le comprendra, l'invention permet de réaliser une aspersion efficace des récipients à stériliser à l'aide d'une rampe unique.

## Revendications

1. Autoclave de stérilisation (10) comprenant une cuve (12) dans laquelle est reçu au moins un conteneur (20) ajouré où sont placés des récipients (22) à stériliser, et au moins une rampe (24) d'aspersion située dans la cuve et destinée à la répartition d'un liquide de stérilisation, lequel se rassemble après ruissellement en partie basse (28) de la cuve, caractérisé en ce qu'il comprend un tambour (14) dans ladite cuve, monté rotatif autour d'un axe (XX) horizontal et recevant ledit conteneur (20), et que ladite rampe (24) s'étend dans un espace délimité entre le tambour (14) et la partie supérieure de la cuve.

2. Autoclave selon la revendication 1, caractérisé en ce que ledit tambour (14) comporte plusieurs goulottes (34) faisant office de godets régulièrement réparties à sa périphérie, ouvertes sensiblement en direction de l'axe de rotation et traversant la partie basse de la cuve au cours de la rotation du tambour.

3. Autoclave selon la revendication 2, caractérisé en ce que lesdites goulottes (34) faisant office de godets ont une forme semi-tubulaire.

4. Autoclave selon l'une quelconque des revendications 1 à 3, caractérisé en ce qu'il comporte une unique rampe (24) d'aspersion.

FIG.1

0243213

FIG.2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 313 847 (STOCK)<br>* Figures 1,4; page 7, lignes 11-26; revendications 1,6 * | 1,4 | A 61 L   2/06<br>A 23 L   3/14 |
| A | AU-A-  27 472  (R.F. HOWELL)<br>--- | | |
| A | DE-C-  666 096  (F. OLDENBURG)<br>--- | | |
| A | US-A-4 225 626  (FMC CORP.)<br>--- | | |
| A | FR-A-2 379 990  (FMC CORP.)<br>--- | | |
| A | US-A-2 629 312  (H.C. DAVIS)<br>--- | | |
| A | DE-A-2 724 174  (A. WEISS)<br>----- | | |

**DOMAINES TECHNIQUES RECHERCHES (Int. Cl.4)**

A 23 L
A 23 B
A 23 C

Le présent rapport de recherche a été établi pour toutes les revendications

| Lieu de la recherche | Date d'achèvement de la recherche | Examinateur |
|---|---|---|
| LA HAYE | 10-08-1987 | GUYON R.H. |

OEB Form 1503 03 82

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant